# EUROPEAN PATENT SPECIFICATION

(11) **EP 1 239 902 B1**
(45) Date of publication and mention of the grant of the patent: **06.07.2005**
(21) Application number: 00975845.9
(22) Date of filing: 21.11.2000
(51) Int. Cl.: A61M 5/24, A61M 5/315

(54) **INJECTION DEVICE**
INJEKTIONSVORRICHTUNG
DISPOSITIF D'INJECTION

(30) Priority: 09.12.1999 DK 176499; 21.12.1999 DK 183299
(43) Date of publication of application: 18.09.2002
(73) Proprietor: NOVO NORDISK A/S, 2880 Bagsvaerd (DK)
(72) Inventor: AASMUL, Soeren, DK-2840 Holte (DK); CHRISTENSEN, Lars, Hofmann, DK-4040 Jyllinge (DK); LJUNGGREEN, Henrik, DK-2850 Ballerup (DK); MUNK, Jens, DK-3650 Oelstykke (DK); HANSEN, Steffen, DK-3400 Hilleroed (DK)
(86) International application number: PCT/DK2000/000643
(87) International publication number: WO 2001/041838

(56) References cited:
- US-A- 4 950 246
- US-A- 5 042 977
- US-A- 5 827 232
- US-A- 5 938 642

## Description

The invention relates to handheld injection devices by which set doses of a liquid medicament can be injected from a medical reservoir by release of a power reservoir in the device.

Said medical reservoir may e.g. be cylinder ampoule comprising a first and a second end of which the first end may be closed by a pierceable membrane which can be pierced by a first end of an injection needle when this needle is mounted on the device, the other end of the injection needle being sharp to pierce the skin where an injection is going to be made. The second end of the ampoule is closed by a piston which can be forced into the ampoule to press out some the content thereof through the needle.

Such devices may be thin and elongated and have the shape of a fountain pen or they may be relative short and thick. Not withstanding the length or thickness, the devices have a distal end at which an injection needle can be mounted and a proximal end from which an injection button projects at least when a dose has been set by a dose setting mechanism, e.g. a button which can be rotated relative to rest of the device. When the projecting injection button is pressed into the device the set dose is injected.

With this construction the device prompts the user to grip with his hand around the device so that the needle projects from the little finger side of the hand and the thumb rests on the injection button so that the force necessary to press this injection button to inject a predetermined amount of medicine from the ampoule is provided by means of the thumb. Whereas this gnp is suited to ensure that a good firm pressure can be exerted on the injection button another kind of gnp is preferred when the needle is going to be inserted through the skin. By this insertion a pencil grip is preferred, i.e. the device is gripped as if it was a pencil and the needle was the lead. After the insertion the grip has to be shifted to the above-described encircling grip, i.e. the grip must be chanced dunng the time when the needle is inserted into the skin. This will be unpleasant and may give a risk of bending or breaking the needle.

It is an objective of the invention to provide a handheld injection device by which this inconvenience is avoided.

This is obtained by a handheld injection device by which set doses of a liquid medicament can be injected fully or partially from a medical reservoir by release of a power reservoir in the device. Said device is characterised in that a release button by activation of which the power reservoir is released is placed in the distal half, preferably in the distal third, of the length of the injection device. When the device is gripped at the distal half of its length with a pencil grip between the thumb and the index finger and the long finger the index finger can easily be moved to press the release button without the grip has to be changed.

The power reservoir may be a battery from which a motor may be energized when a release button is activated or the power reservoir may be a cocked spring that can, when released e.g. by drawing a pawl out of engagement, force a set dose of medicine from the medical reservoir out through an injection needle.

From US 4 950 246 is known a syringe by which a set dose is injected when a button at the proximal end of the syringe is operated to energise a motor in the syringe.

US 5 042 977 describes a syringe wherein a spnng powered injection sequence is started is started when a tngger is operated having the shape of a dip at the proximal end of the syringe is operated.

These known syringes appear to be designed for the traditional encircling gnp with the hand and operation of a button at the proximal end of the syringe although the medicine out pressing force does not have to be delivered by the finger which operates the trigger or injection button.

When the syringe is held as you hold a pencil it is easy for the user to guide the needle carefully into the skin and when the injection release button is placed in the distal half or preferably the distal third of the syringe it can easily without changing the grip be reached by one of the fingers gripping the pencil.

In an appropriate embodiment of the device according to the invention at least part of the distal third of the device may have a cross section, which is ergonomic shaped to be gripped as a pencil is gripped by a thumb, an index finger and a long finger. This ergonomic shape may ensure that the pen is always held in the same rotational position so that the display is facing the user and the release button lies at a position, which makes it easy to reach it by the index finger.

Where the injection force is provided by a motor that can be energised from a battery the release button can be an electric switch turning on the energising of the motor. When the injection force is provided by a cocked spring maintained in its cocked position by a pawl, this pawl may be released by means of a connection between the release button and said pawl when the release button is activated.

In the following the invention is described in further details with references to the drawing, wherein
- Figure 1: shows a handheld injection device according to the invention
- Figure 2: shows the device in figure 1 held with a pencil grip.
- Figure 3: shows schematically an embodiment of an injection device according to the invention,
- Figure 4: shows an exploded picture of the device according to figure 3,
- Figure 5: shows a sectional view of the device according to figure 3, and
- Figure 6: shows another sectional view of the device in figure 3.

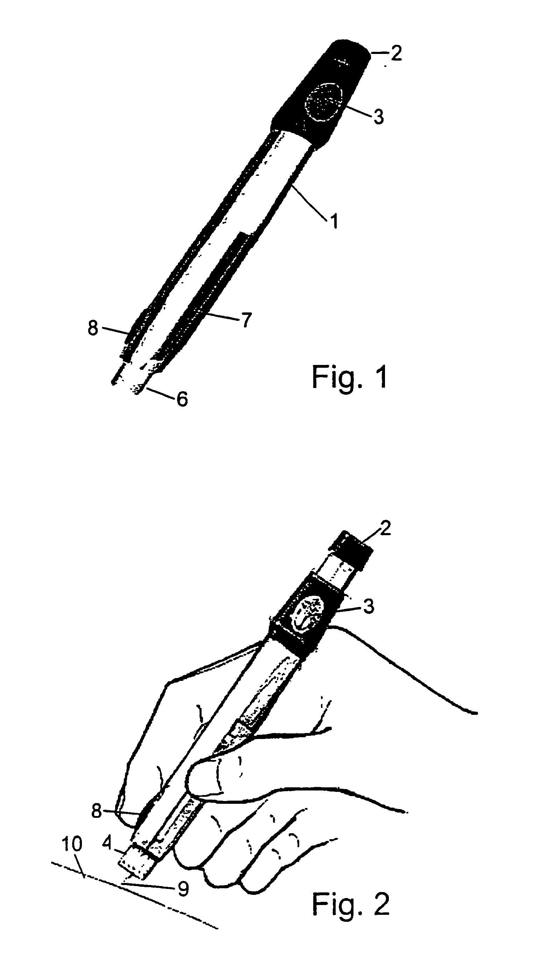

The injection device shown in figure 1 comprises a housing 1, which has at its proximal end a dose-setting button 2 which can be rotated to set a dose. The size of the set dose is shown in a display 3. In its distal half the device accommodates an ampoule with a medicine to be injected by means of the device. The ampoule can be inspected through a window 7 in the housing 1. The ampoule is at its distal end provided with a socket 6 which projects from the distal end of the device and onto which a needle hub with an injection needle can be mounted.

Figure 2 shows the device in figure 1 held by a hand in a pencil grip, i.e. with its distal end held between the index finger and the thumb of a hand and resting on the long finger. The dose-setting button 2 is shown elevated from the proximal end of the device. This indicates that a dose has been set. Further a needle hub 4 with a needle 9 is mounted at the distal end of the device and the needle 9 is inserted through the skin 10 ready for making an injection.

The injection can now be realised by pressing the release button 8 to release a power reservoir in the proximal part of the device.

The power reservoir may be an electric battery by which an electric motor may be energised when a switch is closed by pressing the button 8. Alternatively the power reservoir may be mechanical, e.g. a spring which is cocked and maintained in cocked condition by a detent mechanism, which detent mechanism may be released when the button 8 is pressed. As the power reservoir and the driving mechanism will usually be situated in the proximal half of the device a connection is established to this proximal half of the device from the button 8 in the distal half of said device. Where the power reservoir is an electric battery the connection may be electric leads connecting a switch operated by the release button with the battery and the motor so that the motor is energised when said switch is closed. If the power reservoir is a mechanical one, which is released by withdrawing a detent, the connection from the release button to the detent may be a longitudinal rod that transmits movement of the button 8 to the detent.

The overall cross section of the device and consequently the cross section of the distal half of this device is in the figure shown as being circular, but a not round cross section may be provided at the distal part to give this part an ergonomic shape which makes it fit the long, the index, and the thumb finger gripping it. Hereby the device may be held in a rotational position, which ensures that the display 3 faces the eyes of the user and that a release button 8 by which the injection is triggered lies in a position in which the user can easily reach and operate it by his index finger.

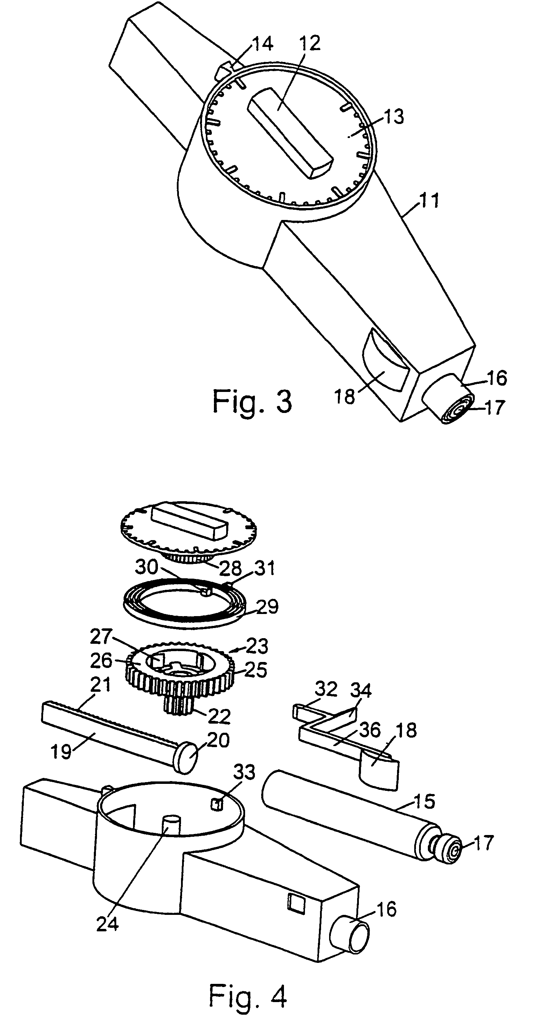

Figure 3 shows schematically an injection device having a housing 11. A dose is set by operating a dose setting grip 12 by which a dial 13 forming the display can be rotated to set a dose the size of which is indicated by a pointer 14 pointing at a scale at the perimeter of the dial. By the setting of a dose a spring inside the housing is cocked and is maintained ready to inject the set dose when a release button 18 is operated. At its distal end the hosing is provided with a socket 16 onto which a needle hub can be mounted, which needle hub has an injection needle projecting from the mounted hub and a back needle which penetrates a sealing 17 at the distal part of an ampoule accommodated in the housing.

Figure 4 shows an exploded picture of the device in figure 3. In this picture is shown an ampoule 15 which is accommodated in the housing 11 with its neck part placed inside the socket 16 with its sealing 17 free to receive the back needle of a needle unit mounted by its needle hub onto the socket 16. A piston rod 19 has a pressure foot 20, which presses on a piston in the ampoule when the piston rod is forced in a distal direction by a drive mechanism compnsing a toothing 21 on the piston rod 19, which toothing is engaged by a pinion 22 on a dnver wheel 23 which is rotatably mounted on a pivot pin 24 in the housing 11. The driver wheel 23 has an annular nm 26 along the outer perimeter of which detent teeth 25 are provided and at an inner perimeter of which pawls 27 are provided. The pawls 27 cooperate with a pawl wheel 28 which is integral with a disk 35 which is integral with the dial, so that the dial 13 with the disk 35 and the pawl wheel 28 can be rotated clockwise relative to the driver wheel 23 but will rotate this driver wheel 23 when the dial is rotated in an anticlockwise direction. When the driver wheel 23 is rotated anticlockwise the pinion 22 will move the piston rod into the ampoule. Also the dial 13 with the disk 35 and the pawl wheel 28 is mounted on the pivot pin 24 and a spiral spring 29 having an inner 30 lug and an outer lug 31 is positioned surrounding the disk 35 between the driver wheel 23 and the dial with its inner lug 30 engaging a recess in disk 35 and its outer lug 31 engaging a hook shaped protrusion 33 on an inner wall of the housing 11.

Rotating the dial 13 clockwise relative to the housing 11 sets a dose and the inner end of the spring 29 will be carried with the dial 13 whereas the outer lug will be fixed relative to the housing 11. This way the spring 29 will be cocked and will try to rotate the dial back to its start position. When the dial is rotated back to its start position it will due to the engagement between the pawls 27 and the pawl wheel 28 try to rotate the driver wheel 23 in an anticlockwise direction. Such a rotation is barred by a detent 32 engaging the teeth 25 of the dnver wheel. 23. The detent 32 is by a rod 36 connected to the release button 18 so that operation of this button can draw the detent 32 out of its engagement with the teeth 25 and this way allow the driver 23 to be rotated by the spring 29 in the anticlockwise direction. A branch 34 on the connecting rod 36 act as a spring which forces the detent 32 into engagement with the teeth 25 of the driver wheel 23 when the release button 18 is not operated.

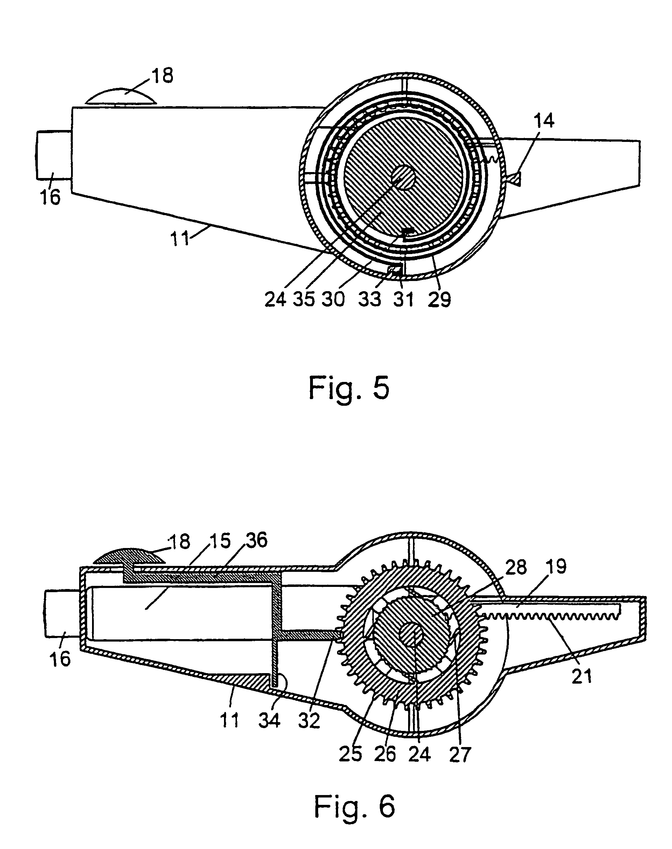

Figure 5 shows a sectional view of the device in figure 3 the just behind the dial 13 to illustrate the engagement of the end lugs 30 and 31 of the spnng 29 with the disk 35 and the hook shaped protrusion 33.

Figure 6 shows another sectional view through the release button 18 and the connecting rod 36. This figure shows how the detent 32 engages the teeth 25 of the driver wheel 23 and how the detent by the spnng 24 is forced into engagement with said teeth of the driver wheel. Further the pawl connection between the pawls 27 of the driver wheel 23 and the toothed pawl wheel 28 of the dial is shown.

## Claims

1. A handheld injection device by which set doses of a liquid medicament can be injected from a medical reservoir (15) through an injection needle (9) mounted to the injection device at a distal end thereof by release of a power reservoir in the device, **characterised in that**, the power reservoir is released fully or partially by activation of a release button (18), placed in the distal half of the length of the injection device and wherein the power reservoir is a cocked spring (29) maintained In its cocked position by a detent (32) which spring (29) when released can press out a set dose of medicine from the medical reservoir (15) through the injection needle (9), and wherein the release button (18) is connected to the detent (32) to release it when said release button (18) is actuated.

2. A handheld injection device with a display (3) by which device set doses of a liquid medicament can be injected from a medical reservoir (15) through an injection needle (9) mounted to the injection device at a distal end thereof by release of a power reservoir in the device, **characterised in that**, the power reservoir is released fully or partially by activation of a release button (8) placed in the distal half of the length of the injection device and wherein the power reservoir is an electric battery by which a motor can be energized to press out a set dose of medicine from the medical reservoir (15) through the injection needle (9) and that the release button (8) is an electric switch by actuation of which the energising of the motor can be switched on, and wherein the release button (8) and the display (3) is located in an interrelated position in which a user can operate the release button (8) with the display (3) facing the eyes of the user.

## Patentansprüche

1. Tragbare Injektionsvomchtung, bei der bestimmte Mengen eines flüssigen Arzneimittels aus einem medizinischen Behälter (15) durch eine Injektionsnadel (9) die an der Injektionsvorrichtung an einem distalen Ende davon befestigt ist, bei Auslösung eines Energiespeichers in der Vorrichtung injiziert werden können, **dadurch gekennzeichnet, dass** der Energiespeicher vollständig oder teilweise durch Aktivierung eines Auslöseknopfes (18), der distal in der Mitte der Länge der Injektionsvorrichtung platziert ist, ausgelöst wird, und wobei der Energiespeicher eine gespannte Feder (29) ist, die mittels einer Sperre (32) in ihrer gespannten Position gehalten wird, deren Feder (29) bei Auslösung bestimmte Mengen des Arzneimittels aus dem medizinischen Behälter (15) durch die Injektionsnadel (9) herauspresst, und wobei der Auslöseknopf (18) mit der Sperre (32) verknüpft ist, um sie auszulösen, wenn der Auslöseknopf (18) betätigt wird.

2. Tragbare Injektionsvomchtung mit einem Display (3), bei welcher Vorrichtung bestimmte Mengen eines flüssigen Arzneimittels aus einem medizinischen Behälter (15) durch eine Injektionsnadel (9) die an der Injektionsvorrichtung an einem distalen Ende davon befestigt ist bei Auslösung eines Energiespeichers in der Vorrichtung injiziert werden können, **dadurch gekennzeichnet, dass** der Energiespeicher vollständig oder teilweise durch Aktivierung eines Auslöseknopfes (8), der distal in der Mitte der Länge der Injektionsvorrichtung platziert ist, ausgelöst wird, und wobei der Energiespeicher eine elektrische Batterie ist, bei der ein Motor zum Herauspressen einer bestimmten Dosis des Medikaments aus dem medizinischen Behälter (15) durch die Injektionsnadel (9) betätigt werden kann, und wobei der Auslöseknopf (8) eine elektrische Schaltung ist, bei deren Betätigung der Motor angetrieben werden kann, und wobei der Auslöseknopf (8) und das Display (3) voneinander abhängig so angebracht sind, dass der Benutzer den Auslöseknopf (8) bedienen kann, während das Display (3) seinen Augen zugewandt ist.

## Revendications

1. Un appareil manuel d'injection par lequel des doses données d'un médicament liquide peuvent être injectées depuis un réservoir médical (15) au travers d'une aiguille d'injection (9) montée sur l'appareil d'injection à une extrémité distale de celui-ci par libération d'une réserve d'énergie dans l'appareil, **caractérisé en ce que** la réserve d'énergie est libérée en tout ou partie par activation d'un bouton de libération (18), placé dans la moitié distale de la longueur de l'appareil d'injection et où la réserve d'énergie est un ressort armé (29) maintenu en position armée par une détente (32), ressort (29) qui lorsqu'il est libéré peut expulser une dose données de médicament depuis le réservoir médical (15) au travers de l'aiguille d'injection (9), et où le bouton de libération (18) est relié à la détente (32) pour la libérer lorsqu'est actionné ledit bouton de libération (18).

2. Un appareil manuel d'injection avec un cadran (3) par lequel des doses données par l'appareil d'un médicament liquide peuvent être injectées depuis un réservoir médical (15) au travers d'une aiguille d'injection (9) montée sur l'appareil d'injection à une extrémité distale de celui-ci par libération d'une réserve d'énergie dans l'appareil, **caractérisé en ce que** la réserve d'énergie est libérée en tout ou partie par activation d'un bouton de libération (8) placé dans la moitié distale de la longueur de l'appareil d'injection et où la réserve d'énergie est une pile électrique par laquelle un moteur peut être alimenté pour expulser une dose données de médicament depuis le réservoir médical (15) au travers de l'aiguille d'injection (9), et **en ce que** le bouton de libération (8) est un interrupteur électrique par l'actionnement duquel l'alimentation du moteur peut être mise en marche, et où le bouton de libération (8) et le cadran (3) sont placés en un position mutuelle où un utilisateur peut actionner le bouton de libération (8) avec l'afficheur (3) face aux yeux de l'utilisateur.
